# EUROPEAN PATENT SPECIFICATION

(11) **EP 2 213 321 B1**
(45) Date of publication and mention of the grant of the patent: **23.07.2014**
(21) Application number: 08854476.2
(22) Date of filing: 19.11.2008
(51) Int. Cl.: A61M 11/00, A61M 15/00, B05B 7/16

(54) **AEROSOL INHALING SYSTEM**
AEROSOLINHALATIONSSYSTEM
SYSTÈME D'INHALATION D'AÉROSOL

(30) Priority: 29.11.2007 JP 2007308710; 29.11.2007 JP 2007308712
(43) Date of publication of application: 04.08.2010
(73) Proprietor: Japan Tobacco Inc., Tokyo 105-8422 (JP)
(72) Inventor: YAMADA, Manabu, Tokyo 130-8603 (JP); KATAYAMA, Kazuhiko, Tokyo 130-8603 (JP); SASAKI, Hiroshi, Tokyo 130-8603 (JP)
(74) Representative: Isarpatent
(86) International application number: PCT/JP2008/071015
(87) International publication number: WO 2009/069518

(56) References cited:
- EP-A1- 0 692 273
- EP-A2- 0 326 174
- JP-A- 11 089 551
- JP-A- 2007 209 789
- JP-T- 2003 530 980
- US-A- 4 284 089
- US-A1- 2002 112 723
- US-A1- 2003 226 837
- US-A1- 2005 268 911
- US-B1- 6 845 771

## Description

### Technical Field

The present invention relates to an aerosol inhalation system for feeding a user with medical products, items of taste and the like in an aerosol form.

### Background Art

An aerosol inhalation system of this type is disclosed, for example, in Kohyo (National Publication of Translated Version) No. 2000-510763. The system disclosed in the publication has a supply pump for supplying a solution (liquid substance) that is a source of aerosol. The supply pump is connected to a pipe. The pipe has an open end, and is filled inside with a solution supplied from the supply pump. A mouthpiece is disposed adjacently to the pipe end of the pipe. An electric heater is arranged so as to surround the pipe end portion of the pipe. The electric heater heats and evaporates the solution existing in the pipe end portion. The vapor of the solution is spontaneously emitted from the pipe end. Such solution vapor is condensed to produce aerosol when exposed to air that is inhaled by the user through the mouthpiece. In this way, the user can inhale the aerosol together with the intake air.

### Disclosure of the Invention

In the case of the system disclosed in the above-mentioned publication, the solution vapor is spontaneously emitted from the pipe end not only during the user's inhalation but also at other times. The spontaneous emission of the vapor wastes the solution, and moreover often empties the pipe end portion immediately before the user starts to inhale. Even if the supply pump is activated in conjunction with the user's inhalation, it takes time until the pipe end portion is refilled with solution, and the solution vapor is spontaneously emitted from the pipe end. As a result, there is a delay in response from the user's inhalation to the generation of aerosol.

It is an object of the invention to provide an aerosol inhalation system that prevents a wasteful use of solution and a delay in generation of aerosol, and also allows a user to inhale the aerosol with ease.

### Means for Solving the Problem

The above-mentioned object is achieved by an aerosol inhalation system of the present invention. The aerosol inhalation system comprises an inhalator including an outside air inlet located in a front end portion thereof, a mouthpiece disposed in a rear end portion of the inhalator, and an aerosol-generating passage extending therein from the outside air inlet to the mouthpiece; a flexible storage bag prepared separately from the inhalator and containing a solution that is a source of aerosol; a feed pipette for leading the solution in the liquid storage bag to the aerosol-generating passage of the inhalator, the feed pipette having a feed opening opened in the aerosol-generating passage and allowing the solution to be sucked from the feed opening into the aerosol-generating passage by an intake airflow when air in the aerosol-generating passage is inhaled through the mouthpiece; and a heating element disposed in the inhalator away from the feed opening, for heating and atomizing the solution sucked from the feed opening into the aerosol-generating passage, and generating aerosol within the aerosol-generating passage.

With the aerosol inhalation system, when a user inhales the air in the aerosol-generating passage through the mouthpiece, this generates an intake airflow traveling from the outside air inlet to the mouthpiece in the aerosol-generating passage. The intake airflow sucks the solution in the feed pipette out of the feeding opening into the aerosol-generating passage. The sucked-out solution is transferred in the direction of the mouthpiece together with the intake airflow. In this transfer process, the solution is evaporated by the heat from the heating element, and generates aerosol in the intake airflow. Thus, the user can speedily inhale the aerosol with the intake air.

In this case, the heating element aerosolizes only the solution sucked out from the feed opening. The solution in the feed pipette, therefore, is prevented from being exposed to heat from the heating element and being volatilized from the feed opening when there is no inhalation by the user.

Specifically, the inhalator further includes an air pipe with the outside air inlet, and the feed pipette is connected into the air pipe through the feed opening. In this case, it is preferable that the heating element should be disposed between the air pipe and the mouthpiece, and be a tubular electric heater defining a part of the aerosol-generating passage therein.

Preferably, the aerosol-generating passage includes a narrow portion reducing a sectional area thereof, and the feed opening opens in an inner surface of the narrow portion. Such a narrow portion increases the velocity of the intake airflow passing through the narrow portion, and thus facilitates the suction of the solution from the feed opening.

When the heating element is an electric heater, the aerosol inhalation system of the invention may further comprise a power source for supplying power to the heater; a power source switch for the power source; a temperature sensor for detecting heater temperature; an indicator for indicating a state of a temperature rise of the heater; and a control circuit for controlling the power supplied to the heater according to the power source switch and a signal from the temperature sensor, maintaining the heater temperature at predetermined operating temperature, and simultaneously activates the indicator.

Desirably, the power source switch, the indicator and the control circuit are assembled into a single control unit, and this control unit is mounted on the inhalator.

With the above-described aerosol inhalation system, the user can inhale through the mouthpiece after confirming the activation of the indicator. At this time, since the heater has already reached the operating temperature, the solution sucked out from the feed opening of the feed pipette is quickly heated and turned into aerosol.

The aerosol inhalation system of the invention may further comprise transportable energy source for supplying the heating element with energy for heating the solution, and a connector for detachably and mechanically connecting the energy source and the heating element to each other and allowing the energy supply from the energy source to the heating element while connecting the energy source to the heating element.

In this aerosol inhalation system, prior to the use of the inhalator, the heating element of the inhalator is mechanically connected to the energy source through the connector. The heating element is thus supplied with energy from the energy source, and the temperature of the heating element is increased to predetermined temperature, or more specifically, an upper limit of an operating temperature range. After the temperature rise of the heating element is completed, the inhalator is detached from the energy source side at the connector. In this state, the user can inhale aerosol in the same manner by using the inhalator.

Being mechanically detachable from the energy source as mentioned, the inhalator is small and lightweight. The inhalator is accordingly easy to handle, which enables the user to inhale the aerosol with ease.

To be specific, the energy source includes a power source for supplying electric energy. In this case, the heating element is a tubular electric heater supplied with the electric energy from the power source to generate heat and forming a part of the aerosol-generating passage therein. Preferably, the aerosol inhalation system may further comprise an electric storage device electrically connected to the heater and chargeable through the connector. The electric storage device supplies power to the heater after the detachment of the inhalator from the power source, and thus increases the duration of use of the inhalator.

The energy source may be a heat source for supplying thermal energy. In this case, the heating element is a thermal storage tube defining a part of the aerosol-generating passage therein, and the connector is a heat coupler for transmitting the thermal energy from the heat source to the thermal storage tube.

The aerosol inhalation system of the invention may further comprise a valve interposed in the feed pipette. In this case, it is preferable that the valve should be opened only in condition that the user inhales. For this reason, as long as the valve is closed, even if the user accidentally inhales or exhales, the valve prevents the suction of the solution from the feed opening into the aerosol-generating passage and the inflow of air into the liquid storage bag.

### Technical Advantage of the Invention

As described above, according to the aerosol inhalation system of the invention, when the user inhales through the mouthpiece, the solution in the feed pipette is sucked out from the feed opening into the aerosol-generating passage. The sucked-out solution is exposed to heat from the heating element to be evaporated, and immediately generates aerosol. This dramatically reduces a response time from the user's inhalation to the aerosol generation.

When there is no inhalation by the user, the solution is not volatilized from the feed opening, so that a waste of the solution is effectively prevented.

Since the inhalation and the solution supply bag are two separate parts, the inhalation is designed to be small and lightweight, and the user can inhale the aerosol with ease.

Other advantages of the aerosol inhalation system of the invention will be explained in the description of embodiments with reference to the attached drawings.

### Brief Description of the Drawings

FIG. 1 is a schematic view showing a system of a first embodiment;
FIG. 2 is an enlarged sectional view showing the inside of an air pipe of FIG. 1;
FIG. 3 is a schematic view showing a system of a second embodiment;
FIG. 4 is a view of the air pipe of FIG. 3 as viewed into an outside air inlet thereof;
FIG. 5 is a schematic view showing a system of a third embodiment;
FIG. 6 is an elevation view of an orifice member of FIG. 5;
FIG. 7 is a schematic view showing a system of a fourth embodiment;
FIG. 8 is a sectional view showing a front end portion of a heater of FIG. 7 in an enlarged scale;
FIG. 9 is a view showing a check valve interposed in a feed pipette;
FIG. 10 is a view showing an electromagnetic valve interposed in the feed pipette;
FIG. 11 is a control block diagram for the system of the invention;
FIG. 12 is a view showing that a control unit of FIG. 11 is mounted onto an inhalator;
FIG. 13 is a schematic view showing a system of a fifth embodiment;
FIG. 14 is a schematic view showing a system of a sixth embodiment;
FIG. 15 is a schematic view showing a system of a seventh embodiment; and
FIG. 16 is a schematic view showing a system of an eighth embodiment.

### Best Mode for Carrying out the Invention

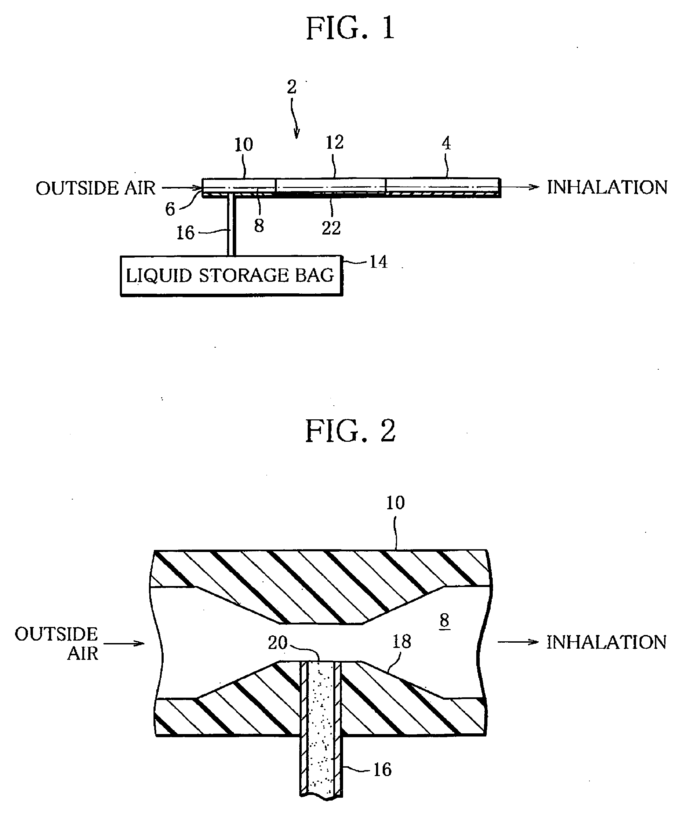

FIGS. 1 and 2 show an aerosol inhalation system (hereinafter, referred to as a system) of a first embodiment.

The system includes an inhalator 2. The inhalator 2 as a whole has the shape of a rod. The inhalator 2 has a front end portion, a middle portion and a rear end portion. The rear end portion is formed into a mouthpiece 4. The inhalator 2 further has an outside air inlet 6 in a front end face thereof. The front end portion and the middle portion define an aerosol-generating passage 8 therein. The aerosol-generating passage 8 extends from the outside air inlet 6 to the mouthpiece 4.

More specifically, the front end and middle portions of the inhalator 2 are made up of an air pipe 10 having the outside air inlet 6 and a tubular heating device, namely, a tubular electric heater 12, respectively. An inner path inside the air pipe 10 and the heater 12 is formed as the aerosol-generating passage 8.

A flexible liquid storage bag 14 is prepared outside the inhalator 2. The liquid storage bag 14 is filled with a solution that is a source of aerosol. To be concrete, the solution is medical products, items of taste or the like. The items of taste may include tobacco constituents.

A feed pipette 16 extends from the liquid storage bag 14. The liquid storage bag 14 is detachably connected to the aerosol-generating passage 8, or the air pipe 10, of the inhalator 2 through the feed pipette 16. Specifically, the feed pipette 16 is connected to the aerosol-generating passage 8 at a connect position that is located at predetermined distance away from the heater 12 in the direction of the outside air inlet 6. The feed pipe 16 leads the solution in the liquid storage bag 14 to the aerosol-generating passage 8 due to a capillary phenomenon.

Preferably, as illustrated in FIG. 2, the air pipe 10 includes a narrow portion 18 therein. The narrow portion 18 is provided with a feed hole connecting the air pipe 10 to the outside. An one end portion of the feed pipette 16 is inserted in the feed hole. The one end of the feed pipe 16 is formed as a feed opening 20 for opening in an inner surface of the narrow portion 18. The feed pipette 16 has a connector (not shown) in the other end thereof. Through this connector, the feed pipette 16 is detachably connected to the liquid storage bag 14.

The first end of the feed pipette 16 may be detachably connected to the feed hole through a connector (not shown). In this case, the feed opening 20 is formed by the feed hole.

According to the present embodiment, the heater 12 is a ceramic heater, but is not limited to the ceramic heater. Preferably, the heater 12 has an outer circumferential surface covered with a heat-resistant thermal barrier coating 22.

In the above-described system according to the first embodiment, the heater 12 of the system is supplied with power before the user inhales air in the aerosol-generating passage 8 through the mouthpiece 4. Thus, the temperature of the heater 12 is increased to predetermined operating temperature and maintained at this operating temperature.

In this state, when the user inhales as described, outside air enters from the outside air inlet 6 and flows into the aerosol-generating passage 8. This outside air inflow produces an intake airflow traveling from the outside air inlet 6 towards the mouthpiece 4 within the aerosol-generating passage 8. The intake airflow makes pressure around the feed opening 20 in the aerosol-generating passage 8 into negative pressure in relation to pressure in the feed pipette 16. Since the feed opening 20 is disposed in the inner surface of the narrow portion 18, this increases difference, or negative pressure, between the pressure in the feed pipette 16 and the pressure around the feed opening 20. As a result, when the user inhales, the solution in the feed pipette 16 is sucked out through the feed opening 20 into the aerosol-generating passage 8. The sucked-out solution is then transferred to the heater 12 together with the intake airflow. The solution is then heated by the heater 12 to be evaporated into the intake air, and is condensed by the intake air to be turned into aerosol.

This makes short a response time from the beginning of the user's inhalation through the suction of the solution to the aerosol generation. Even if there is no inhalation by the user while the heater 12 is maintained at the operating temperature, the solution existing at the feed opening 20 is not volatilized by heat from the heater 12 since the feed pipette 16 and the connect position of the feed pipette 16 with respect to the aerosol-generating passage 8, that is, the feed opening 20, are located at predetermined distance away from the heater 12. Consequently, the solution in the feed pipette 16 is not consumed at other times than the user's action for feeding the solution, which reliably prevents a waste of the solution.

Since the inhalator 2 does not include the liquid storage bag 14 therein, the inhalator 2 may be formed to have a rod-shaped appearance similar to a rod-shaped smoking article, such as a filter cigarette and a cigar. For the same reason, the inhalator 2 is lightweight. Even if the liquid storage bag 14 has a high capacity, the user can handle the inhalator 2 as easily as with the filter cigarette or the cigar, and can inhale the aerosol by means of the inhalator 2 without difficulty.

The invention is not restricted by the first embodiment. The other embodiments will be described below. In the description of the other embodiments, members and components having similar functions to those of the previous embodiment(s) will be provided with the same reference marks, and the description of such members and components will be omitted.

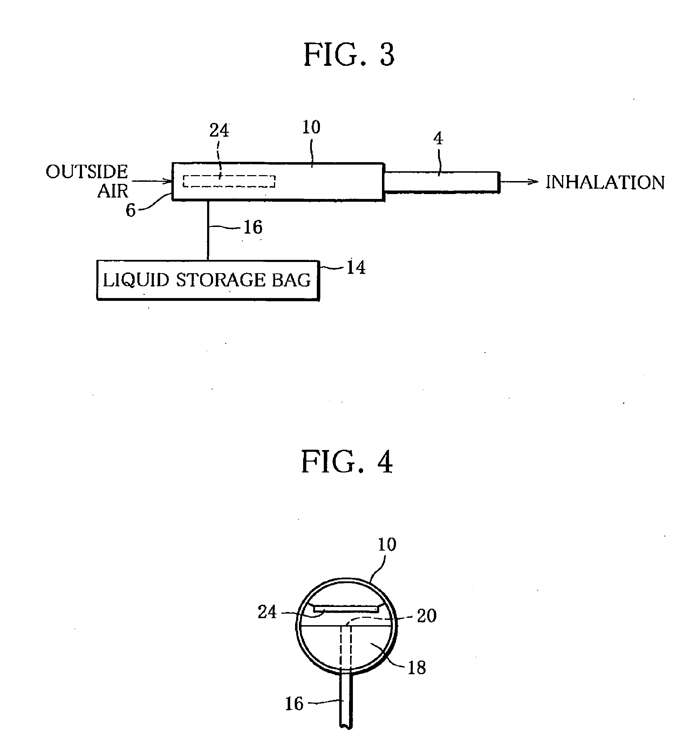

FIGS. 3 and 4 show a system of a second embodiment.

In the second embodiment, the inhalator 2 has the air pipe 10 and the mouthpiece 4. The air pipe 10 and the mouthpiece 4 are directly connected to each other without the heater 12 to be interposed therebetween. A plate-like electric heater 24 is contained in the air pipe 10. The electric heater 24 is also a ceramic heater. The electric heater 24 is disposed at predetermined distance away from the feed opening 20 of the feed pipette 16, and is arranged along the aerosol-generating passage 8 within the air pipe 10. To be specific, the electric heater 24 has an upstream end portion facing to the feed opening 20, and extends from the feed opening 20 towards the mouthpiece 4 over predetermined length.

In the system of the second embodiment, too, the user's inhalation sucks the solution out of the feed opening 20 into the air pipe 10, and the sucked-out solution is transferred towards the mouthpiece 4 together with an intake airflow. In this transfer process, the solution is evaporated by the heat from the electric heater 24, and aerosol is thus generated in the intake airflow.

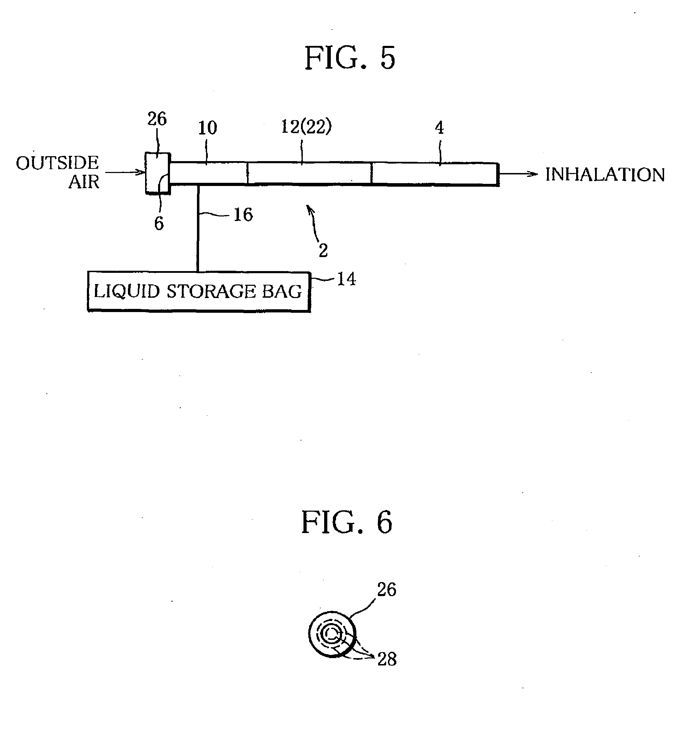

FIGS. 5 and 6 show a system of a third embodiment.

The system of the third embodiment differs from that of the first embodiment only in that a ring-shaped orifice member 26 is detachably fitted to the front end of the air pipe 10, namely, the outside air inlet 6. The orifice member 26 has an orifice 28 smaller than a diameter of the outside air inlet 6. During the user's inhalation, the orifice 28 regulates an intake rate of the outside air flowing into the aerosol-generating passage 8 through the outside air inlet 6, and determines negative pressure produced near the feed opening 20. In other words, the opening degree of the orifice 28 determines the amount of the solution sucked out from the feed opening 20 of the feed pipette 16, or aerosol generation amount, per inhalation by the user.

It is therefore desirable that the opening degree (diameter) of the orifice member 26 should be variable as shown by a chain double-dashed line in FIG. 6. In this case, the amount of the solution sucked out from the feed opening 20 (aerosol generation amount) per inhalation by the user can be adjusted by changing the opening degree of the orifice member 26.

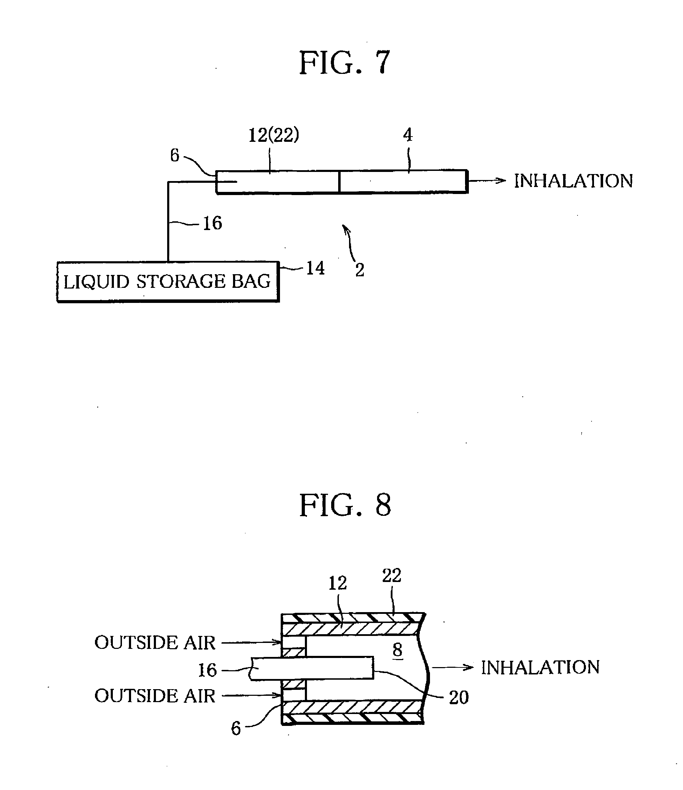

FIGS. 7 and 8 show a system of a fourth embodiment.

In the fourth embodiment, the inhalator 2 has the heater 12 and the mouthpiece 4 connected to the heater 12. A front end of the heater 12 is formed as the outside air inlet 6. In this case, the aerosol-generating passage 8 is defined only by an inner path of the heater 12.

The feed pipette 16 has the feed opening 20 inserted from the outside air inlet 6 into the heater 12 and opens towards the mouthpiece 4 within the heater 12. More specifically, as is apparent from FIG. 8, the front end of the heater 12 is formed as a closed end, and the feed pipette 16 penetrates through the center of the closed end. In this embodiment, the outside air inlet 6 is made up of a plurality of circular or semi-circular apertures. These apertures are so distributed as to surround the feed pipette 16.

In the system of the fourth embodiment, too, the user's inhalation sucks the solution out of the feed opening 20 of the feed pipette 16 into the heater 12, or the aerosol-generating passage 8. The sucked-out solution contacts the inner surface of the heater 12. At this point of time, the solution is quickly heated by the heater 12. Aerosol is thus generated in an intake airflow and then inhaled by the user with the intake airflow.

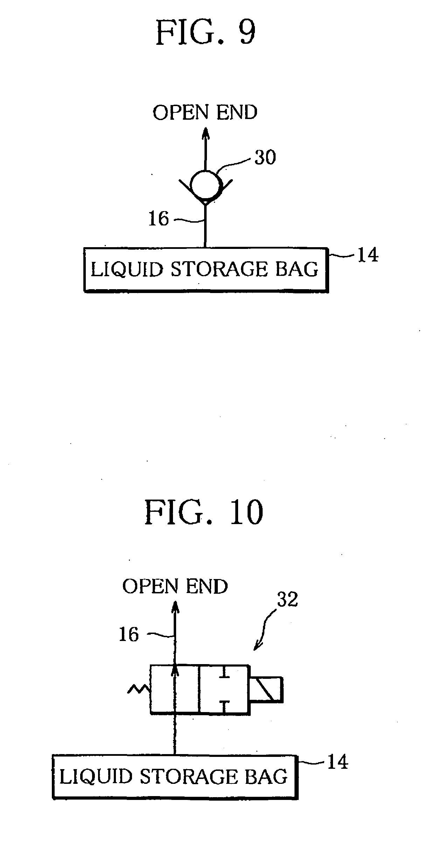

In the systems of the first to fourth embodiments, as illustrated in FIG. 9 or 10, the feed pipette 16 may have a check valve 30 or an electromagnetic valve 32. The check valve 30 allows the solution to travel only in the direction from the liquid storage bag 14 through the feed pipette 16 towards the feed opening 20, and inhibits the opposite flow of the solution. Because of this, even if the user exhales into the aerosol-generating passage 8 through the mouthpiece 4, the solution in the feed pipette 16 does not flow into the liquid storage bag 14 with air, and the air is firmly prevented from flowing into the liquid storage bag 14.

The electromagnetic valve 32 has a similar function to that of the check valve 30. Moreover, as long as kept at a closed position, the electromagnetic valve 32 reliably prevents the solution from being sucked out from the feed opening 20 even if the user accidentally inhales before the heater 12 reaches the operating temperature.

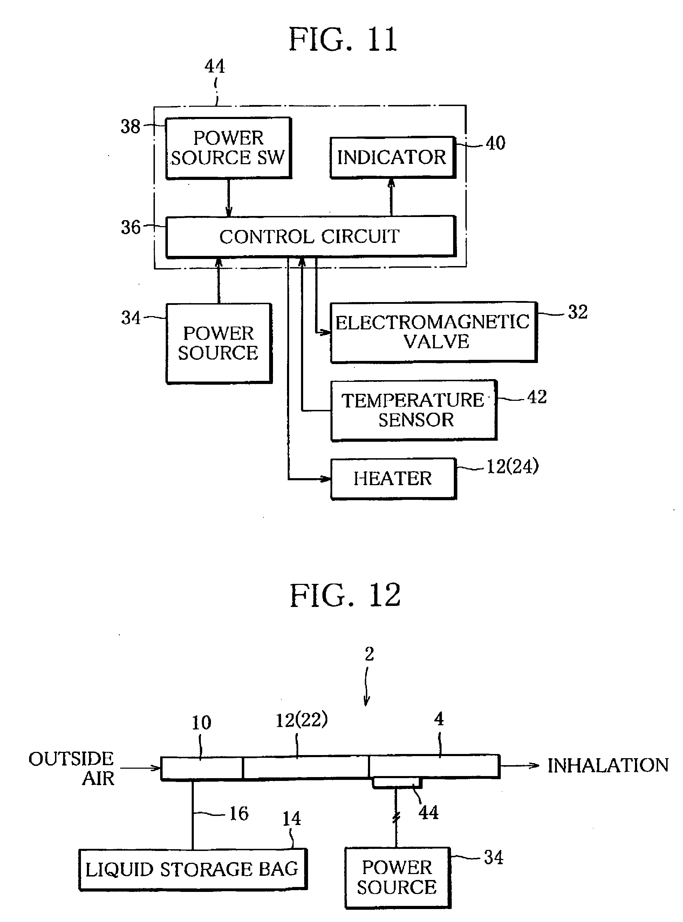

FIG. 11 is a control block diagram of the systems of the first to fourth embodiments.

The systems each have a power source 34, such as a cell, which supplies power to the heater 12 (or 24). The power source 34 is electrically connected to a power source switch 38 through a control circuit 36. Electrically connected to the control circuit 36 is not only the heater 12 (or 24) but also an indicator 40, such as an LED, and a temperature sensor 42 for detecting the temperature of the heater 12 (or 24). In the system of the fourth embodiment, the electromagnetic valve 32 is also electrically connected to a control circuit 36.

When the power source switch 38 is turned on by the user, the control circuit 36 starts the power supply from the power source 34 to the heater 12 (or 24), and thus increases the temperature of the heater 12 (or 24) towards the operating temperature. After the temperature of the heater 12 (or 24) reaches the operating temperature, the control circuit 36 controls the power supply to the heater 12 (or 24) according to a detection signal from the temperature sensor 42, and maintains the temperature of the heater 12 (or 24) at the operating temperature.

When the heater 12 (or 24) is maintained at the operating temperature, the control circuit 36 activates the indicator 40 and informs the user of the completion of the temperature rise of the heater 12 (or 24).

The user can then inhale the aerosol without fail by making an inhaling action after confirming the activation of the indicator 40. To put it differently, the indicator 40 contributes to prevent the user's accidental inhalation in the process of temperature rise of the heater 12 (or 24).

With the control circuit 36, it is also possible to make the electromagnetic valve 32 open and close in response to ON and OFF operations of the power source switch 38. However, it is preferable in the control circuit 36 that the electromagnetic valve 32 should be opened and closed in response to ON and OFF operations of the indicator 40. In this case, the electromagnetic valve 32 is closed while the power source switch 38 is off or while the heater 12 (or 24) is in the process of temperature rise. As a result, even if the user accidentally inhales, the solution is not sucked out from the feed opening 20 of the feed pipette 16.

The control circuit 36, the power source switch 38, and the indicator 40 are assembles into a single control unit 44 as enclosed by a dashed line in FIG. 11. The control unit 44 is attached, for example, onto an outer surface of the mouthpiece 4 as illustrated in FIG. 12. Since the control unit 44 attached onto the mouthpiece 4 does not contain the power source 34, the control unit 44 is lightweight and is easily reduced in size.

The inhalator 2 can therefore maintain the rod-shaped appearance similar to a smoking article, such as a filter cigarette and a cigar, in spite of the control unit 44. This allows the user to easily hold the inhalator 2 between his/her fingers and inhale the aerosol by using the inhalator 2 with ease.

The temperature sensor 42 shown in FIG. 11 may be replaced with a current sensor (not shown). The current sensor detects the current supplied from the power source 34 to the heater 12 (or 24). On the basis of a result of the detection, the control circuit 36 estimates whether or not the heater 12 (or 24) reaches the operating temperature.

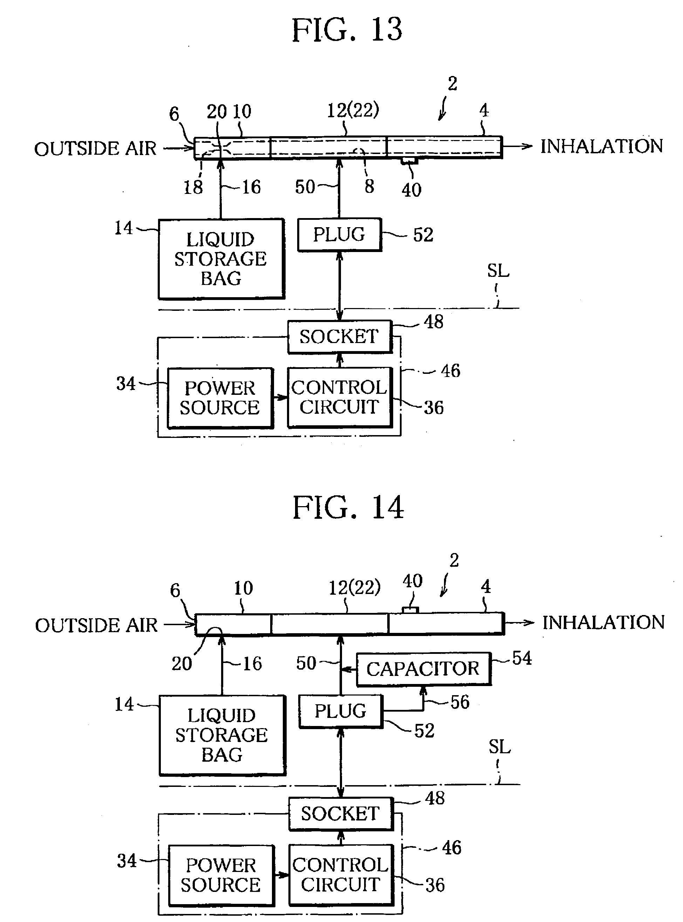

FIG. 13 shows a system of a fifth embodiment.

In the fifth embodiment, the power source 34 and the control circuit 36 are assembled into a single transportable power unit 46. The power unit 46 has a socket 48. The indicator 40 is attached to the mouthpiece 4 of the inhalator 2. The indicator 40, the temperature sensor 42 and the heater 12 are connected to a plug 52 through electric lines. Only an electric code 50 connecting the heater 12 to the plug 52 is shown in FIG. 13.

The plug 52 is able to insert into the socket 48 of the power unit 46. The socket 48 and the plug 52 enable the power unit 46 and the inhalator 2 to be mechanically and electrically connected to each other, and allow the detachment of the inhalator 2 from the power unit 46. Reference mark SL in FIG. 13 represents a detachment line between the inhalator 2 and the power unit 46.

When the plug 52 is inserted into the socket 48, power is supplied from the power source 34 through the control circuit 36 to the heater 12. The temperature of the heater 12 is increased towards an upper limit of an operating temperature range. In short, the socket 48 and the plug 52 has the above-mentioned function of the power source switch 38.

The control circuit 36 then determines whether or not the temperature of the heater 12 has been increased to the upper limit on the basis of the detection signal from the temperature sensor 42. If the result is YES, the control circuit 36 activates the indicator 40 and informs the user that the inhalator 2 is ready to use.

The user can pull the plug 52 of the inhalator out of the socket 48 of the power unit 46 after confirming the activation of the indicator 40, and can inhale the aerosol by means of the inhalator 2 in the above-described manner in a state where the inhalator 2 is detached from the power unit 46.

In the fifth embodiment, during the use of the inhalator 2, the heater 12 is detached from the power source 34, and therefore cannot be supplied with power. As a result, after the plug 52 is pulled out of the socket 48, the temperature of the heater 12 is gradually decreased as time passes. However, as long as the temperature of the heater 12 is kept within the operating temperature range, the heater 12 is capable of generating aerosol in response to the user's inhalation.

In the fifth embodiment, after the plug 52 of the inhalator 2 is pulled out of the socket 48 of the power unit 48 after the heater 12 is once heated, the indicator 40 stops operating. It is then impossible for the user to know whether the temperature of the heater 12 is within the operating temperature range while using the inhalator 2. In order to solve this problem, the indicator 40 may include therein a retaining circuit (not shown) for retaining the operation of the indicator 40 on the basis of the detection signal from the temperature sensor 42 while the temperature of the heater 12 is within the operating temperature range.

FIG. 14 shows a system of a sixth embodiment.

The system of the sixth embodiment further has a capacitor 54 serving as an electric storage device. The capacitor 54 is disposed in a charging cord 56. The charging cord 56 electrically connects between a power source cord 50, which is arranged between the plug 52 and the heater 12, and the plug 52. When the plug 52 is inserted into the socket 48, and the temperature of the heater 12 is increased, the capacitor 54 stores the electric energy supplied from the power source 4.

For that reason, even if the inhalator 2 is used in a state mechanically and electrically disconnected from the power unit 46, the temperature of the heater 12 is not decreased since power is supplied from the capacitor 54 to the heater 12. Consequently, as compared to the system of the fifth embodiment, the system of the sixth embodiment is capable of increasing the duration of use of the inhalator 2 per process of the temperature rise of the heater 12.

FIG. 14 shows a system of a seventh embodiment.

The system of the seventh embodiment further has a thermostatic valve 58. The valve 58 is interposed in the feed pipette 16. Specifically, the valve 58 includes a valve element made of shape-memory alloy. The valve element is thermally connected to the heater 12 through a heat-transfer line 60. In this case, the valve 58 is opened only when the temperature of the heater 12 is within the operating temperature range. The valve 58 is closed when the temperature of the heater 12 drops lower than the operating temperature range.

Since the valve 58 closes the feed pipette 16 when the temperature rise of the heater 12 is not completed, the user's accidental inhalation does not suck the solution out of the feed opening 20 of the feed pipette 16 into the aerosol-generating passage 8. That is to say, the temperature of the heater 12 is kept within the operating temperature range when the user's inhalation sucks the solution out of the feed opening 20, so that all the solution that has been sucked out is turned into aerosol.

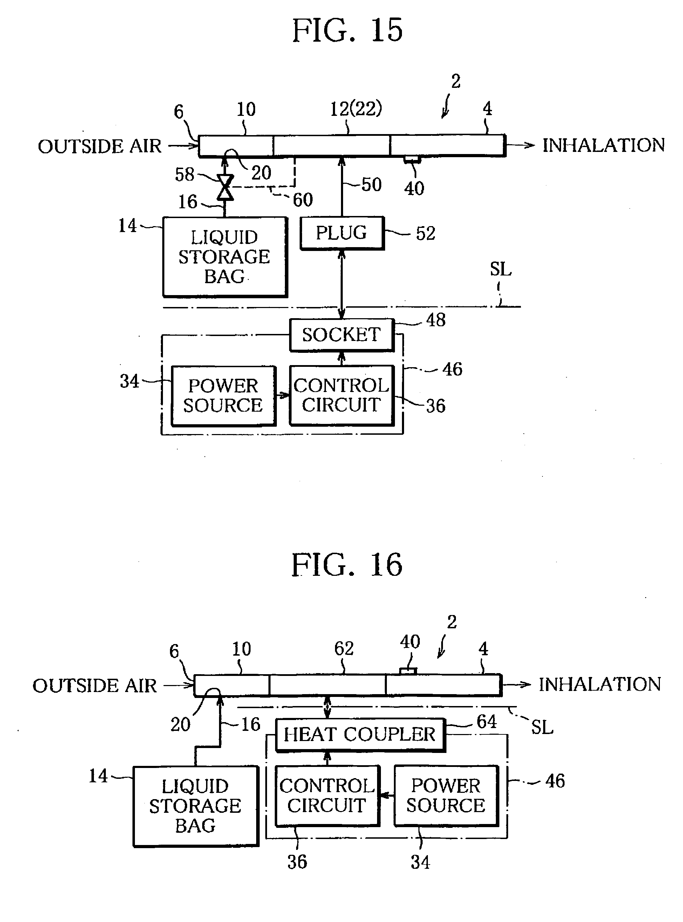

FIG. 16 shows a system of an eighth embodiment.

In the system of the eighth embodiment, the inhalator 2 has a thermal storage tube 62, instead of the heater 12. The power unit 46 includes a heat coupler 64, instead of the socket 48. The heat coupler 64 has such a shape as to be able to receive the inhalator 2 at the thermal storage tube 62. To be concrete, the heat coupler 64 includes an electric heater (not shown) therein. When a switch (not shown) of the power unit 46 is turned on, the power source 34 supplies power to the electric heater of the heat coupler 64 through the control circuit 36, and heats the heat coupler 64 by using the electric heater thereof. The inhalator 2 is received by the heat coupler 64 at the thermal storage tube 62 thereof, and if the heat coupler 64 is heated in this state, thermal energy is transmitted from the heat coupler 64 to the thermal storage tube 62. As a result, the temperature of the thermal storage tube 62 is increased to the upper limit of the operating temperature range. Thereafter, the user detaches the inhalator 2 from the heat coupler 64, and inhales aerosol by means of the inhalator 2 in the same manner as described above.

In the fifth to eighth embodiments, the socket 48 or the heat coupler 64 may be a separate body from the power unit 46.

Lastly, the invention is not limited to the systems of the first to eighth embodiments. The invention is limited by the appended claims.

## Claims

1. An aerosol inhalation system comprising:
an inhalator (2) including an outside air inlet (6) located in a front end portion thereof, a mouthpiece (4) disposed in a rear end portion thereof, and an aerosol-generating passage(8) extending therein from the outside air inlet (6) to the mouthpiece (4);
a flexible liguid storage bag (14) prepared separately from said inhalator (2) and containing a solution that is a source of aerosol;
a feed pipette (16) for leading the solution in said liquid storage bag (14) to the aerosol-generating passage (8) of said inhalator (2), said feed pipette (16) having a feed opening (20) opened in the aerosol-generating passage (8), for allowing the solution to be sucked from the feed opening (20) into the aerosol-generating passage (8) by an intake airflow when air in the aerosol-generating passage (8) is inhaled through the mouthpiece (14); and
a heating element (12) disposed in said inhalator (2) away from the feed opening (20), said heating element (12) for heating and atomizing the solution sucked from the feed opening (20) into the aerosol-generating passage (8), and generating aerosol within the aerosol-generating passage (8).

2. The aerosol inhalation system according to claim 1, wherein said inhalator (2) further includes an air pipe (10) with the outside air inlet (6), and said feed pipette (16) is connected into the air pipe (10) through the feed opening (20).

3. The aerosol inhalation system according to claim 2, wherein said heating element (12) is disposed between the air pipe (10) and the mouthpiece (4), and is a tubular electric heater defining a part of the aerosol-generating passage (8) therein.

4. The aerosol inhalation system according to claim 1, wherein the aerosol-generating passage (8) includes a narrow portion (18) reducing a sectional area thereof, and the feed opening (20) opens in an inner surface of the narrow portion (18).

5. The aerosol inhalation system according to claim 1, wherein:
said heating element includes an electric heater (12);
the system further comprises:
a power source (34) for supplying power to said heater (12);
a power source switch (38) for said power source (34);
a temperature sensor (42) for detecting heater temperature;
an indicator (40) for indicating a state of a temperature rise of said heater (12); and
a control circuit (36) for controlling the power supplied to said heater (12) according to the power source switch (38) and a signal from said temperature sensor (42), maintaining the heater temperature at predetermined operating temperature, and simultaneously activating said indicator (40).

6. The aerosol inhalation system according to claim 5, wherein said power source switch (38), said indicator (40) and said control circuit (36) are assembled into a single control unit (44), and said control unit (44) is mounted on said inhalator (2).

7. The aerosol inhalation system according to claim 1 further comprising a transportable energy source (46) for supplying said heating element (12) with energy to heat the solution, and a connector (48, 52) for detachably and mechanically connecting said energy source (46) and said heating element (12) to each other, and allowing the energy supply from said energy source (46) to said heating element (12) while connecting said energy source (46) to said heating element (12).

8. The aerosol inhalation system according to claim 7, wherein:
said energy source (46) includes a power source (34) for supplying electric energy; and
said heating element (12) is a tubular electric heater supplied with the electric energy from the power source (34) to generate heat and forms a part of the aerosol-generating passage (8).

9. The aerosol inhalation system according to claim 8, further comprising an electric storage device (54) electrically connected to said heater (12), said electric storage device (54) being chargeable through said connector.

10. The aerosol inhalation system according to claim 7, wherein:
said energy source (46) is a heat source for supplying thermal energy;
said heating element is a thermal storage tube (62) defining a part of the aerosol-generating passage (8); and
said connector is a heat coupler (64) for transmitting the thermal energy from said heat source to said thermal storage tube (62).

11. The aerosol inhalation system according to claim 1, further comprising a valve (58) interposed in said feed pipette (16).

12. The aerosol inhalation system according to claim 11, wherein said valve (58) is opened only on condition that the user inhales.

## Patentansprüche

1. Aerosolinhalationssystem, das Folgendes umfasst:
- einen Inhalator (2), der einen Außenlufteinlass (6) umfasst, der sich an seinem Vorderendabschnitt befindet, ein Mundstück (4), das in seinem Hinterendabschnitt angeordnet ist, und einen Aerosol-generierenden Durchgang (8), der sich darin von dem Außenlufteinlass (6) zu dem Mundstück (4) erstreckt;
- einen flexiblen Flüssigkeitsspeicherbeutel (14), der separat von dem Inhalator (2) ausgebildet ist und eine Lösung enthält, die eine Aerosolquelle ist;
- eine Zuführpipette (16) zum Führen der Lösung in dem Flüssigkeitsspeicherbeutel (14) zu dem Aerosol-generierenden Durchgang (8) des Inhalators (2), wobei die Zuführpipette (16) eine Zuführöffnung (20) aufweist, die in den Aerosol-generierenden Durchgang (8) hinein öffnet, damit die Lösung durch einen Ansaugluftstrom aus der Zuführöffnung (20) in den Aerosol-generierenden Durchgang (8) gesaugt werden kann, wenn Luft in dem Aerosol-generierenden Durchgang (8) durch das Mundstück (14) eingeatmet wird; und
- ein Heizelement (12), das in dem Inhalator (2) von der Zuführöffnung (20) entfernt angeordnet ist, wobei das Heizelement (12) dazu dient, die Lösung, die aus der Zuführöffnung (20) in den Aerosol-generierenden Durchgang (8) gesaugt wird, zu erwärmen und zu zerstäuben und ein Aerosol innerhalb des Aerosol-generierenden Durchgangs (8) zu bilden.

2. Aerosolinhalationssystem nach Anspruch 1, wobei der Inhalator (2) des Weiteren ein Luftrohr (10) mit dem Außenlufteinlass (6) umfasst und die Zuführpipette (16) durch die Zuführöffnung (20) mit dem Luftrohr (10) verbunden ist.

3. Aerosolinhalationssystem nach Anspruch 2, wobei das Heizelement (12) zwischen dem Luftrohr (10) und dem Mundstück (4) angeordnet ist und eine röhrenförmige elektrische Heizung ist, die darin einen Teil des Aerosol-generierenden Durchgangs (8) definiert.

4. Aerosolinhalationssystem nach Anspruch 1, wobei der Aerosol-generierende Durchgang (8) einen schmalen Abschnitt (18) umfasst, der seinen Querschnitt verkleinert, und die Zuführöffnung (20) in eine Innenfläche des schmalen Abschnitts (18) hinein öffnet.

5. Aerosolinhalationssystem nach Anspruch 1, wobei:
das Heizelement eine elektrische Heizung (12) umfasst;
das System des Weiteren Folgendes umfasst:
- eine Stromquelle (34) zum Zuführen von Strom zu der Heizung (12);
- einen Stromquellenschalter (38) für die Stromquelle (34) ;
- einen Temperatursensor (42) zum Detektieren der Heizungstemperatur;
- einen Indikator (40) zum Anzeigen eines Zustands eines Temperaturanstiegs der Heizung (12); und
- einen Steuerkreis (36) zum Steuern des der Heizung (12) zugeführten Stroms gemäß dem Stromquellenschalter (38) und einem Signal des Temperatursensors (42), Halten der Heizungstemperatur auf einer zuvor festgelegten Betriebstemperatur und gleichzeitiges Aktivieren des Indikators (40).

6. Aerosolinhalationssystem nach Anspruch 5, wobei der Stromquellenschalter (38), der Indikator (40) und der Steuerkreis (36) zu einer einzigen Steuereinheit (44) zusammengesetzt sind und die Steuereinheit (44) an dem Inhalator (2) montiert ist.

7. Aerosolinhalationssystem nach Anspruch 1, das des Weiteren eine tragbare Energiequelle (46) umfasst, um das Heizelement (12) mit Energie zu versorgen, um die Lösung zu erwärmen, und einen Verbinder (48, 52) umfasst, um die Energiequelle (46) und das Heizelement (12) lösbar und mechanisch miteinander zu verbinden und es zu ermöglichen, dass die Energie von der Energiequelle (46) zu dem Heizelement (12) fließen kann, während die Energiequelle (46) mit dem Heizelement (12) verbunden ist.

8. Aerosolinhalationssystem nach Anspruch 7, wobei:
die Energiequelle (46) eine Stromquelle (34) zum Zuführen von elektrischer Energie umfasst; und
das Heizelement (12) eine röhrenförmige elektrische Heizung ist, die mit der elektrischen Energie von der Stromquelle (34) beaufschlagt wird, um Wärme zu erzeugen, und einen Teil des Aerosol-generierenden Durchgangs (8) bildet.

9. Aerosolinhalationssystem nach Anspruch 8, das des Weiteren eine elektrische Speichervorrichtung (54) umfasst, die elektrisch mit der Heizung (12) verbunden ist, wobei die elektrische Speichervorrichtung (54) durch den Verbinder geladen werden kann.

10. Aerosolinhalationssystem nach Anspruch 7, wobei:
die Energiequelle (46) eine Wärmequelle zum Zuführen von Wärmeenergie ist;
das Heizelement eine Wärmespeicherröhre (62) ist, die einen Teil des Aerosol-generierenden Durchgangs (8) definiert; und
der Verbinder ein Wärmekoppler (64) zum Übertragen der Wärmeenergie von der Wärmequelle zu der Wärmespeicherröhre (62). ist

11. Aerosolinhalationssystem nach Anspruch 1, das des Weiteren ein Ventil (58) umfasst, das in der Zuführpipette (16) angeordnet ist.

12. Aerosolinhalationssystem nach Anspruch 11, wobei das Ventil (58) nur unter der Bedingung geöffnet wird, dass der Nutzer einatmet.

## Revendications

1. Système d'inhalation d'aérosol comprenant :
- un inhalateur (2) comprenant une entrée d'air externe (6) située dans une partie d'extrémité avant de celui-ci, un embout buccal (4) disposé dans une partie d'extrémité arrière de celui-ci, et un passage de génération d'aérosol (8) s'étendant dans celui-ci depuis l'entrée d'air externe (6) vers l'embout buccal (4) ;
- un sac de stockage de liquide souple (14) préparé séparément dudit inhalateur (2) et contenant une solution qui est une source d'aérosol ;
- une pipette d'alimentation (16) pour diriger la solution dans ledit sac de stockage de liquide (14) vers le passage de génération d'aérosol (8) dudit inhalateur (2), ladite pipette d'alimentation (16) ayant une ouverture d'alimentation (20) ouverte dans le passage de génération d'aérosol (8), pour permettre à la solution d'être aspirée depuis l'ouverture d'alimentation (20) dans le passage de génération d'aérosol (8) par un écoulement d'air d'admission lorsque de l'air dans le passage de génération d'aérosol (8) est inhalé à travers l'embout buccal (14); et
- un élément chauffant (12) disposé dans ledit inhalateur (2) à distance de l'ouverture d'alimentation (20), ledit élément chauffant (12) pour le chauffage et la pulvérisation de la solution aspirée à partir de l'ouverture d'alimentation (20) dans le passage de génération d'aérosol (8), et la génération d'un aérosol dans le passage de génération d'aérosol (8).

2. Système d'inhalation d'aérosol selon la revendication 1, dans lequel ledit inhalateur (2) comprend en outre un conduit d'air (10) avec l'entrée d'air externe (6), et ladite pipette d'alimentation (16) est reliée dans le conduit d'air (10) à travers l'ouverture d'alimentation (20).

3. Système d'inhalation d'aérosol selon la revendication 2, dans lequel ledit élément chauffant (12) est disposé entre le conduit d'air (10) et l'embout buccal (4), et est un dispositif de chauffage électrique tubulaire définissant une partie du passage de génération d'aérosol (8).

4. Système d'inhalation d'aérosol selon la revendication 1, dans lequel le passage de génération d'aérosol (8) comprend une partie étroite (18) réduisant une surface de section de celui-ci, et l'ouverture d'alimentation (20) débouche sur une surface interne de la partie étroite.

5. Système d'inhalation d'aérosol selon la revendication 1, dans lequel :
ledit élément chauffant comprend un élément chauffant électrique (12) ;
le système comprend en outre .
- une source d'énergie (34) pour alimenter en énergie ledit dispositif de chauffage (12) ;
- un commutateur de source d'alimentation (38) pour ladite source de puissance (34) ;
- une sonde de température (42) pour détecter la température du dispositif de chauffage ;
- un indicateur (40) pour indiquer un état d'une augmentation de la température dudit élément chauffant (12) ; et
- un circuit de commande (36) pour commander la puissance fournie audit élément chauffant (12) en fonction du commutateur de source d'alimentation (38) et un signal provenant dudit capteur de température (42), maintenant la température du dispositif de chauffage à une température de fonctionnement prédéterminée, et activant en même temps ledit indicateur (40).

6. Système d'inhalation d'aérosol selon la revendication 5, dans lequel ledit commutateur de source d'énergie (38), ledit indicateur (40) et ledit circuit de commande (36) sont assemblés dans une seule unité de commande (44), et ladite unité de commande (44) est montée sur ledit inhalateur (2).

7. Système d'inhalation d'aérosol selon la revendication 1, comprenant en outre une source d'énergie transportable (46) pour alimenter ledit élément chauffant (12) avec de l'énergie pour chauffer la solution, et un connecteur (48, 52) pour relier ensemble de manière amovible et mécaniquement ladite source d'énergie (46) et ledit élément chauffant (12), et permettre la fourniture d'énergie à partir de ladite source d'énergie (46) audit élément chauffant (12) tout en reliant ladite source d'énergie (46) audit élément chauffant (12).

8. Système d'inhalation d'aérosol selon la revendication 7, dans lequel :
ladite source d'énergie (46) comprend une source d'alimentation (34) pour fournir de l'énergie électrique; et
ledit élément chauffant (12) est un élément chauffant électrique tubulaire fourni avec l'énergie électrique provenant de la source d'alimentation (34) pour produire de la chaleur et forme une partie du passage de génération d'aérosol (8).

9. Système d'inhalation d'aérosol selon la revendication 8, comprenant en outre un dispositif de stockage électrique (54) relié électriquement audit dispositif chauffant (12), ledit dispositif de stockage électrique (54) pouvant être chargé par l'intermédiaire dudit connecteur.

10. Système d'inhalation d'aérosol selon la revendication 7, dans lequel :
ladite source d'énergie (46) est une source de chaleur pour fournir de l'énergie thermique ;
ledit élément chauffant est un tube de stockage thermique (62) définissant une partie du passage de génération d'aérosol (8) ; et
ledit connecteur est un coupleur de chaleur (64) pour la transmission de l'énergie thermique provenant de ladite source de chaleur vers ledit tube de stockage thermique (62) .

11. Système d'inhalation d'aérosol selon la revendication 1, comprenant en outre un clapet (58) interposé dans ladite pipette d'alimentation (16).

12. Système d'inhalation d'aérosol selon la revendication 11, dans lequel ledit clapet (58) est ouvert seulement à condition que l'utilisateur inhale.
